# EUROPEAN PATENT APPLICATION

(11) **EP 1 972 291 A1**
(43) Date of publication of application: **24.09.2008**
(21) Application number: 08005126.1
(22) Date of filing: 19.03.2008
(51) Int. Cl.: A61B 18/14, A61L 31/10, B05D 7/00

(54) **Non-stick surface coated electrodes and method for manufacturing same**

(30) Priority: 20.03.2007 US 726017
(71) Applicant: Tyco Healthcare Group, LP, North Haven CT 06473 (US)
(72) Inventor: Bastian, Barbara, Boulder, CO 80303 (US)
(74) Representative: HOFFMANN EITLE

(57) **Abstract**

A method of manufacturing an electrosurgical instrument includes the steps of: providing an electrosurgical instrument having at least one conductor, applying a lipid coating onto at least a portion of at least one conductor on an electrosurgical instrument, and curing the lipid coating. An electrosurgical instrument manufactured by the method disclosed herein is also provided.

## Description

### TECHNICAL FIELD

The present disclosure relates to improved electrosurgical instruments and, more particularly, to electrodes having an improved non-stick coating and methods for making same.

### BACKGROUND OF RELATED ART

Electrosurgical instruments, such as vessel sealing instruments, are well known in the medical field for use in sealing vessels and tissue bundles for tissue ligation both in laparoscopic and open surgery applications with concurrent transmission of electrical energy to the contacted tissue. While such surgical tools have proven effective in vessel sealing procedures, problems have been encountered with respect to sticking of tissue to certain portions of the surgical instrument.

In the past, significant efforts have been directed to improvements in electrosurgical instruments and the like, with a view towards providing improved transmission of electrical energy to patient tissue in both an effective manner and to reduce the sticking of soft tissue to the instrument's surface during application. In general, such efforts have envisioned non-stick surface coatings, such as polymeric materials, e.g. polytetrafluoroethylene (PTFE, commonly sold under the trademark TEFLON®) for increasing the lubricity of the tool surface. However, these materials may interfere with the efficacy and efficiency of hemostasis and have a tendency to release from the instrument's substrate due to formation of microporosity, delamination and/or abrasive wear, thus exposing underlying portions of the instrument to direct tissue contact and related sticking issues. In turn, these holes or voids in the coating lead to nonuniform variations in the capacitive transmission of the electrical energy to the tissue of the patient and may create localized excess heating, resulting in tissue damage, undesired irregular sticking of tissue to the electrodes and further degradation of the non-stick coating.

### SUMMARY

A method of manufacturing an electrosurgical instrument with reduced tissue adhesion is provided and includes the steps of: providing an electrosurgical instrument having at least one conductor; applying a lipid coating to at least a portion of the at least one conductor; and curing the lipid coating onto at least a portion of the at least one conductor. The step of curing the lipid coating onto at least a portion of the at least one conductor includes the step of: heating at least one conductor to a temperature in the range from about 140°C to about 160°C for a time period in the range of about 1.5 hours to about 2.5 hours.

The method further includes the step of: repeating the step of applying a lipid coating onto at least a portion of the at least one conductor followed by the step of repeating the step of curing the lipid coating to at least a portion of the at least one conductor wherein the step of curing the lipid coating onto at least a portion of the at least one conductor comprises heating the at least one conductor at least one conductor to a temperature in the range from about 140°C to about 160°C for a time period in the range of about 1.5 hours to about 2.5 hours.

A non-stick electrosurgical instrument is also provided and includes a handle portion; and at least one electrode having a lipid cured to at least a portion of a surface thereof.

### BRIEF DESCRIPTION OF THE DRAWINGS

Various embodiments are described herein with reference to the drawings wherein:
Figure 1 shows an endoscopic forceps for use in an endoscopic procedure;
Figure 2 shows an open forceps for use in open surgical procedures; and
Figure 3 shows an electrosurgical pencil for use in a general surgical procedure.

### DETAILED DESCRIPTION

Disclosed herein is a method of manufacturing an electrosurgical instrument with reduced tissue adhesion properties and generally includes the steps of: providing an electrosurgical instrument having at least one conductor, applying a lipid coating to at least a portion of the conductor, and curing the lipid coating onto the portion of the conductor. The curing step may be carried out utilizing any suitable temperature for any suitable amount of time. However, in some embodiments, thermal curing may take place in stages at temperatures of about 140° C to about 160° C. In other embodiments, curing may take place in stages at temperatures ranging from 145° C to about 155° C, and in further embodiments, curing may take place in temperatures at about 150° C for a period from one hour to three hours. In some embodiments, the time period may vary from about 1.5 hours to 2.5 hours or, in other embodiments, in about 2 hours. In yet other embodiments, the coating may be cured in stages of one to four times per day.

The lipid coating generally comprises multiple coating layers applied to at least a portion of the electrode surface in a sequence for substantially optimized adherence thereto. In some embodiments, the method further includes repeating the step of applying a lipid coating to at least a portion of the conductor(s) followed by repeating the step of curing the lipid coating to at least a portion of the conductor(s). The repeated step of curing the lipid coating to at least a portion of the conductor(s) may include the step of heating the conductor(s) to a temperature in the range from about 130° C. to about 160° C., or in one of the temperature ranges or time periods specified above.

In some embodiments, the surface of the conductor(s) may consist of metal, ceramic or polymeric material that includes at least one electrode. It is contemplated that the lipid coating may decrease the edge effects attributed to delivering RF energy to electrodes having sharp transitions between the conductive electrode and the electrosurgical instrument. Moreover, the lipid coating may reduce the current density along the electrode and instrument interface resulting in a more uniform power density, which reduces the incidence and severity of char and/or coagulum formation. The more uniform current density along the axis of the instrument also results in a more uniform temperature distribution along the electrode. Further, by coating a conductor with lipids to create the outer conductor surface as a subsequent manufacturing step, less labor-intensive methods of forming electrodes and bonding wires to electrodes can be used.

The electrode is an electrically conducting element that is usually elongated and may be in the form of a thin flat blade with a pointed or rounded distal end. Alternatively, the active electrode may include an elongated narrow cylindrical needle that is solid or hollow with a flat, rounded, pointed or slanted distal end. Typically electrodes of this sort are known in the art as "blade", "loop" or "snare", "needle" or "ball" electrodes.

An electrosurgical instrument formed from the presently disclosed method is also provided and includes a handle portion and at least one electrode having a lipid cured to at least a portion of a surface thereof. The non-stick electrosurgical instrument incorporates the lipid coating to cover at least the region thereof utilized to contact a patient's tissue. The coating is designed to provide a low surface energy and low coefficient of friction (high lubricity) with little or no sticking of a patient's tissue to the instrument. The coating is securely bonded to the surface of the instrument for enhanced long term coating stability without delamination for peeling, and further exhibits improved surface hardness for extended wear characteristics.

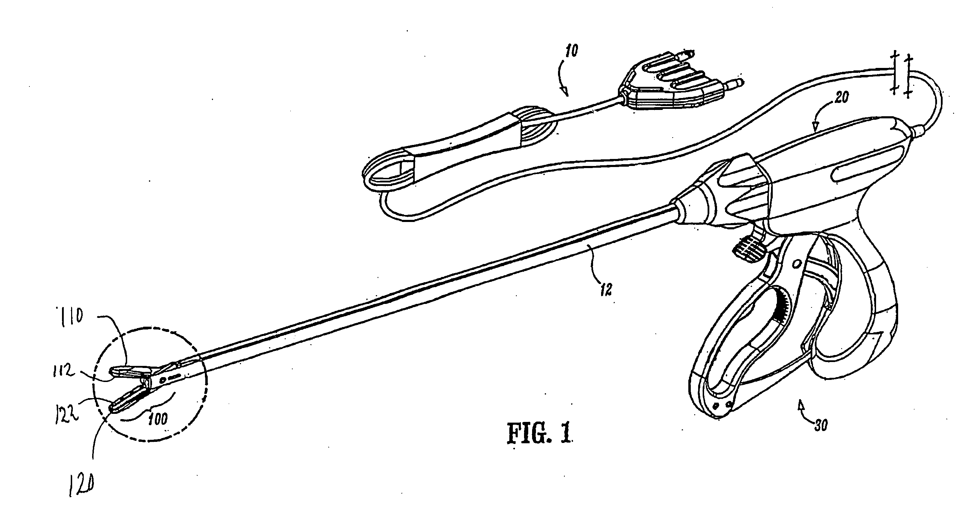

Referring now to the Figures, Figures 1 through 3 show example embodiments of electrosurgical instruments that may be used with the presently disclosed invention. For example, Fig. 1 shows an endoscopic forceps 10 for use with endoscopic procedures. Forceps 10 generally includes a housing 20 having a shaft 12 that extends therefrom. Shaft 12 includes an end effector assembly 100 attached to a distal end thereof that includes jaw members 110 and 120. A handle 30 is included that is operable to move the jaw members from a first position in spaced relation relative to one another to a second position for grasping tissue. Each jaw member 110 and 120, respectively, includes an electrically conductive sealing plate 112 and 122 attached thereto that is disposed in electrical communication with a generator (not shown), which supplies first and second electrical potentials to respective jaw members 110 and 120 for energizing tissue.

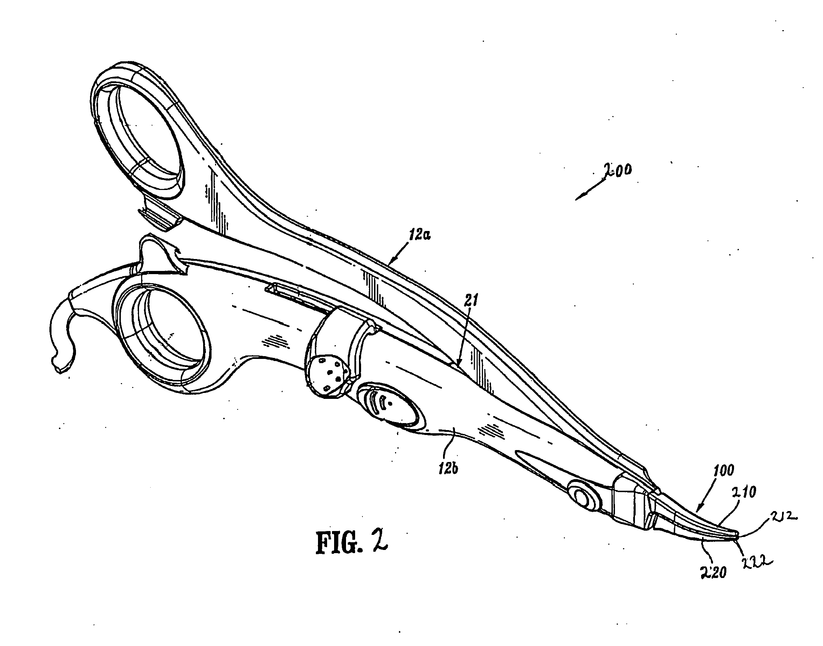

Fig. 2 shows an open forceps 200 for use in open surgical procedures that includes two moveable shaft members 12a and 12b that are configured to actuate a pair of jaw members 210 and 220 disposed at the distal end thereof. The jaw members 210 and 220 are movable from a first position in spaced relation relative to one another to a second position for grasping tissue. Each jaw member 210 and 220, respectively, includes an electrically conductive sealing plate 212 and 222 attached thereto that is disposed in electrical communication with a generator (not shown), which supplies first and second electrical potentials to the respective jaw members for energizing tissue.

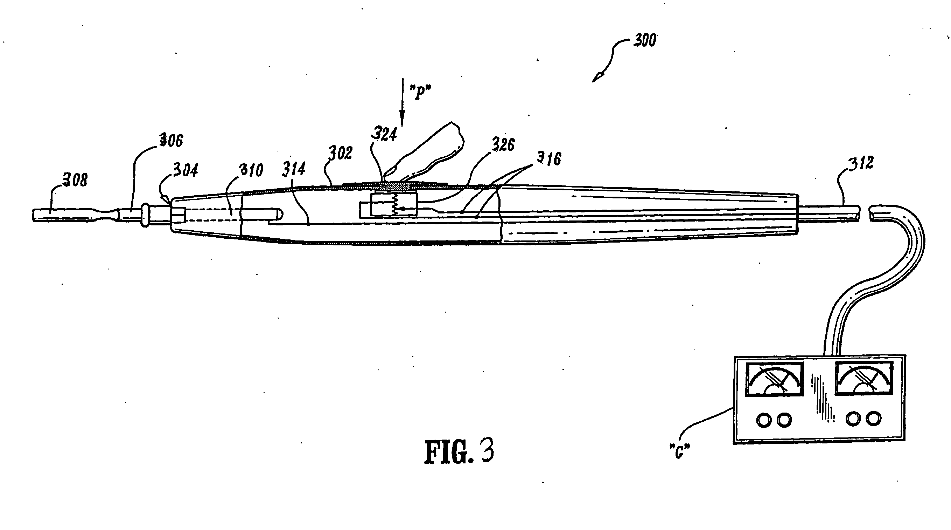

Fig. 3 shows a partially broken, side elevational view of an electrosurgical pencil that is intended to include instruments with a handpiece attached to a selectively removable active electrode (or electrocautery blade) and used to coagulate, cut and/or seal tissue. Referring to Fig. 3 in detail, the electrosurgical monopolar pencil 300 includes an elongated housing 302 configured and adapted to support a blade receptacle 304 at a distal end thereof that, in turn, receives a replaceable electrocautery blade 306 therein. A distal end portion 308 of blade 306 extends distally from receptacle 304 while a proximal end portion 310 of blade 306 is retained within the distal end of housing 302.

As shown, electrosurgical pencil 300 is coupled to a conventional electrosurgical generator "G" via a cable 312. Cable 312 includes a transmission wire 314 that electrically interconnects the electrosurgical generator "G" with the proximal end portion 310 of blade 306. Cable 312 further includes a control loop 316 that electrically interconnects an activation button 324, supported on an outer surface 307 of the housing 302, with the electrosurgical generator "G". Activation button 324 is operatively connected to a pressure transducer 326 (or other variable power switch) that, in turn, controls the RF electrical energy supplied from generator "G" to electrosurgical blade 306.

Although the present disclosure identifies the lipid as being coated onto a portion of the conductor or electrode of an electrosurgical instrument, it will be recognized and understood that the method of the present disclosure may be used in application with other types of electrosurgical instruments, such as electrosurgical needles, blades, pencils and other electrosurgical tools, whether monopolar or bipolar.

In one embodiment, the lipid utilized may be a phospholipid. Phospholipids define a group of phosphate-containing lipids including the major structural lipids of most cellular membranes, e.g., phosphatidyl phospholipids and sphingomyelins. In some embodiments, suitable phospholipids include animal and plant phospholipids; egg phospholipids; soya bean phospholipids or lecithin; com phospholipids; wheat germ, flax, cotton, and sunflower seed phospholipids; milk fat phospholipids; glycerophospholipids; sphingophospholipids; phosphatides; phospholipids containing fatty acid esters including palmitate, stearate, oleate, linoleate, and arachidonate, which esters can be mixtures and mixtures of isomers in the phospholipids; phospholipids composed of fatty acids containing one or more than one double bonds, such as dioleoyl phosphatidylcholine and egg phosphatidylcholine that are not stable as powders but are hygroscopic and can absorb moisture and become gummy; phospholipids composed of saturated fatty acids that are stable as powders and are less amenable to absorption of moisture; glycerides, such as monoglycerides, diglycerides, triglycerides, phosphoglycerides; glycolipids such as glycosphingolipids; phosphatidylserines; phosphatidylcholines; phosphatidylethanolamines; phosphatidylinositols; phosphatidylglycerols such as dimyristoyl phosphatidylglycerol, L-alpha-dimyristoyl phosphatidylglycerol also known as 1,2-dimyristoyl-sn-glycero-3-phospho(rac-1-glycerol) and also known as DMPG; phosphatidic acid; hydrogenated natural phospholipids; and commercially available phospholipids, such as those available from Electrolube®, a division of H.K. Wentworth, Ltd., and/or those available from Cargill, i.e., Leciprime^{™} 1500 Soy Lecithin Fluid.

In some embodiments, suitable phospholipids of the present disclosure include any of various light hydrocarbon oils, such as mineral oil, vegetable oil and the like and any hydrocarbon oils or phospholipids that are approved for human use. In the absence of an internal counterion in the phospholipid, a suitable counterion is a monovalent cation, such as sodium ion. The phospholipid may be salted or desalted, hydrogenated, partially hydrogenated, or unsaturated, natural, synthetic, or semisynthetic.

The lipid may also be provided either as a solution in an organic solvent or, alternatively, as a liposomal or other suspension in an aqueous fluid. The coating may include a phospholipid suspension that may be lyophilised or otherwise dried on the surface. As noted, the formulation may contain components such as phosphatidlycholine and cholesterol adapted to promote liposomal formation on mixing with an aqueous fluid. Generally, however, phosphatidylcholine will not comprise an effective amount of the phospholipid, except in instances where liposome formation is desired. The phospholipid formulation may also contain suitable biologically active materials, including antibiotics and antithrombotic pharmaceuticals.

The lipid coating may be applied in a solvent carrier, which is then evaporated from the surface to concentrate and/or adhere the lipid to the instrument's surface. The lipid coating may also be applied to the surface of the instrument in a polymeric carrier.

The lipid coating is present at a concentration to improve, that is to say, to prevent the adhesion of tissue to metals. Lipid concentrations are in the range of about 5 x 10⁻⁴⁰ to about 5 x 10²⁰ µmol/cm², in embodiments from about 5 x 10²⁰ to about 5 x 10⁻¹⁰ µmol/cm², in embodiments from about 5 x 10⁻¹⁰ to about 500 µmol/cm².

The lipid coating may be applied onto an electrosurgical instrument of the present disclosure utilizing any suitable method, including, for example, dipping, spraying, vapor deposition, brushing, and the like. In some embodiments, the lipid coating is applied with a coating thickness of about 0.5 to about 500 microns, in other embodiments of about 400 to about 4000 microns.

In polymer chemistry and process engineering, curing refers to the toughening or hardening of a polymer material by cross-linking of polymer chains, brought about by chemical additives, ultraviolet radiation, Electron beam (EB), microwave beam (MB), air (i.e., oxygen) or heat via a thermal oven.

In general, a thicker layer of lipid coating will require somewhat longer exposure to heat or UV light than a thinner one, but the relationship is not directly proportional. Also, the rate of cure increases with the amount of UV intensity or heat deposited on the surface-but again the relationship is not directly proportional. The rate of curing may also depend on the distance of the surface of the instrument from the heat source.

In some embodiments, thermal curing is used to adhere the lipid coating to the electrosurgical instrument conductors, e.g. electrodes. Thermal curing has no special features in terms of method but instead takes place in accordance with the customary and known methods, such as heating in a forced air oven or irradiation with IR lamps.

When cured, these lipid coatings, used in thermoset coatings, adhesive/sealants, pottings, and encapsulations, reportedly provide durability, strength, hardness, impact resistance, adhesion, electrical insulation characteristics, and inertness to many chemicals, including water and common solvents.

For optimum adhesion of the lipid coating, substrates must be carefully cleaned before application, especially because of the possible presence of oils, greases, release agents, dirt, and other contaminants. Such cleaning may take place by grit blasting the surface of the electrosurgical instrument surface, by using a plasma cleaner, a sonicator, UV light and/or by dipping the substrate into an acid bath, such as chromic acid, alcohol, chloroform or aqueous solution of a hydroxide of an alkali earth metal followed by washing and rinsing in de-ionized water. Cleaning may also occur by baking at a relatively high temperature of about 400-425°C to volatize the oils, grease and other contaminants prior to application of the lipid.

In many cases, such as with metals and other inorganic substrates, the degree of cleanliness can be ascertained by a simple test that involves spreading a few drops of cool water on the surface. If the water spreads over the area with a continuous film, parts are sufficiently clean for further processing. If the water beads or stays in puddles, EPA acceptable solvents, such as IPA or acetone, should be used for degreasing. The water test should then be repeated before applying the lipid coating.

While the above description contains many specifics, these specifics should not be construed as limitations on the scope of the disclosure, but merely as exemplifications of embodiments thereof. Those skilled in the art will envision many other possibilities within the scope and spirit of the disclosure as defined by the claims appended hereto.

## Claims

1. A method of manufacturing an electrosurgical instrument, comprising the steps of:
providing an electrosurgical instrument having at least one conductor;
applying a lipid coating to at least a portion of the at least one conductor; and
curing the lipid coating.

2. The method according to claim 1, wherein the step of curing the lipid coating includes the step of heating the at least one conductor to a temperature in a range from about 140°C to about 160°C for a time period in a range of about 1.5 hours to about 2.5 hours.

3. The method according to claim 1, wherein the step of curing the lipid coating includes the step of heating the at least one conductor to a temperature at about 150°C and for a time period of about two hours.

4. The method according to any preceding claims, further comprising the step of repeating the step of applying a lipid coating onto at least a portion of the at least one conductor followed by the step of repeating the step of curing the lipid coating.

5. The method according to claim 4, wherein the repeated step of curing the lipid coating includes the step of heating the at least one conductor to a temperature in a range from about 140°C to about 160°C for a period in a range of about 1.5 hours to about 2.5 hours.

6. The method according to claim 4, wherein the repeated step of curing the lipid coating includes the step of heating the at least one conductor to a temperature of about 150°C for a time period of about two hours.

7. The method according to any preceding claim, wherein the at least one conductor includes a surface selected from the group consisting of metal, ceramic and polymeric.

8. The method according to any preceding claim, wherein the at least one conductor includes at least one electrode.

9. The method according to any preceding claim, wherein the lipid is selected from the group consisting of mineral oil, ElectroLube®, lecithin, vegetable oil, monoglycerides, diglycerides, triglycerides, phosphatidylcholine, glycolipids, fatty acids, phospholipids, phosphoglycerides, glycerophospholipids.

10. The method according to any preceding claim, wherein the step of applying comprises a process selected from the group consisting of spray coating, dip coating, vapor deposition, brushing, and combinations thereof.

11. The method according to any preceding claim, wherein the step of curing the lipid coating includes curing via thermal ovens.

12. An electrosurgical instrument, comprising:
a handle portion; and
at least one electrode having a lipid cured to at least a portion of a surface thereof.

13. The electrosurgical instrument according to claim 12, wherein the instrument is at least one of a monopolar instrument and a bipolar instrument.

14. The electrosurgical instrument according to claim 13, wherein the monopolar instrument is a pencil.

15. The electrosurgical instrument according to claim 13, wherein the bipolar instrument is a forceps.
